# EUROPÄISCHE PATENTSCHRIFT

(11) **EP 0 864 646 B1**
(45) Veröffentlichungstag und Bekanntmachung des Hinweises auf die Patenterteilung: **09.08.2006**
(21) Anmeldenummer: 98890036.1
(22) Anmeldetag: 12.02.1998
(51) Int. Cl.: C12N 7/06, A61K 39/12

(54) **Verfahren zur Inaktivierung von lipidumhüllten Viren**
Process for the inactivation of enveloped viruses
Procédé d'inactivation de virus enveloppés de lipides

(30) Priorität: 24.02.1997 AT 29997
(43) Veröffentlichungstag der Anmeldung: 16.09.1998
(60) Teilanmeldung: 05112299.2
(73) Patentinhaber: Baxter Healthcare S.A., 8304 Wallisellen (CH)
(72) Erfinder: Barrett, Noel, 3400 Klosterneuburg/Weidling (AT); Kistner, Otfried, Dr., 1040 Wien (AT); Dorner, Friedrich, Prof., 1230 Wien (AT)
(74) Vertreter: Alge, Daniel

(56) Entgegenhaltungen:
- EP-A- 0 278 487
- WO-A-97/01640
- US-A- 4 164 565
- US-A- 4 314 997
- US-A- 4 673 733
- GROSS P A ET AL: "COMPARISON OF NEW TRITON X-100- AND TWEEN-ETHER-TREATED SPLIT-PRODUCT VACCINES IN CHILDREN" JOURNAL OF CLINICAL MICROBIOLOGY, WASHINGTON, DC, US, Bd. 14, Nr. 5, November 1981 (1981-11), Seiten 534-538, XP000990520 ISSN: 0095-1137
- DANIHELKOVA H ET AL: "DISRUPTION OF INFLUENZA VIRUS A BY DI ETHYL ETHER TWEEN AND TRI-N BUTYL PHOSPHATE TWEEN MIXTURES" ACTA VIROLOGICA (PRAGUE) (ENGLISH EDITION), Bd. 28, Nr. 1, 1984, Seiten 26-32, XP001062455 ISSN: 0001-723X

## Beschreibung

Die Erfindung betrifft ein Verfahren zur Inaktivierung von lipidumhüllten Viren mit einem nicht-ionischen Detergens und die Herstellung eines Impfstoffes enthaltend das inaktivierte Virus. Desweiteren betrifft die Erfindung ein inaktiviertes Virus, das sich durch seine strukturelle Integrität, insbesondere der Hüllproteine, auszeichnet, sowie die Verwendung des inaktivierten Virus zur Herstellung eines Impfstoffes.

Die Entwicklung von effizienten Impfstoffen für die Prophylaxe gegen virale Infektionskrankheiten ist eines der Hauptziele der Medizin in den vergangenen Jahrzehnten. Bei der Herstellung von Lebendvirusvakzinen werden Virusmutanten verwendet, die idente Antigene wie das Wildtypvirus besitzen, jedoch eine reduzierte Pathogenität oder Virulenz aufweisen.

Für die Herstellung von Totimpfstoffen werden Wildtypviren durch physikalische oder chemische Behandlung, etwa mit Formalin, Hydroxylamin, β-Propionlacton oder UV-Strahlen inaktiviert, wobei die Art der Inaktivierung, insbesondere im Hinblick auf die Erhaltung der Immunogenität der viralen Antigene, bedeutsam ist. Die meistverwendete Technik zur Virusinaktivierung bei der Impfstoffherstellung ist die milde Behandlung von Viren mit Formalin, wobei jedoch eine lange Inkubationszeit bis zu 15 Tagen (z.B. bei HAV) erfolgen muß, um eine ausreichende Reduktion der Virusaktivität zu erreichen.

Gesamtvirus-Impfstoffe stimulieren im allgemeinen die Entwicklung von zirkulierenden Antikörpern gegen die Hüllproteine des Virus. Es wird daher immer wieder darauf hingewiesen, daß beim Inaktivierungsprozeß gesichert sein muß, daß die Immunogenität der viralen Proteine erhalten bleibt. Gleichzeitig muß jedoch gesichert sein, daß alle Viren in der Präparation inaktiviert sind, um eine sichere Vakzine zu gewährleisten.

Aufgrund von Zwischenfällen mit unvollständig inaktivierten Gesamtvirus-Impfstoffen oder Kontaminationen der Vakzinepräparation mit zellulären Komponenten oder unerwünschten viralen Partikeln wurde nach alternativen Inaktivierungsverfahren gesucht (Horaud, Develop. Biol. Standard. 75 (1991), 3-7; Brown, Develop. Biol. Standard. 81 (1993), 103-107).

Da eine Formalin-Inaktivierung nicht immer vollständig ist und in einzelnen Vakzine-Präparationen restliche infektiöse Viren festgestellt wurden (Smith et al., Am. J. Hyg. 63 (1956), 150-164) schlugen Mussgay et al. (Intervirology 1 (1973), 259-268) ein zweistufiges Inaktivierungsverfahren vor, wobei eine Formalin-Inaktivierung einer Behandlung mit Tween 80/Ether, NP 40 oder Deoxycholat folgte. Sie stellten jedoch fest, daß durch die zusätzliche Behandlung mit dem Detergens bzw. Lösungsmittel/Detergens die Protektivität der Formalin-inaktivierten Vakzine beeinträchtigt war.

Alternativ zu Impfstoffen enthaltend inaktivierte Gesamtviren erfolgte daher in den letzten Jahren vermehrt die Entwicklung von Subunit-Vakzinen. Bei der Herstellung von Subunit-Vakzinen wird das intakte Virus mit einem starken Detergens solubilisiert, die viralen Proteine aus dem Virus gelöst und selektive Antigene, die in der Lage sind, protektive Antikörper zu stimulieren, isoliert und für die Vakzin-Herstellung verwendet. Gleichzeitig werden nicht-virale Proteine und Membranbestandteile des Virus eliminiert, die möglicherweise unerwünschte Nebenwirkungen bei der Verabreichung der Vakzine haben könnten. Die für die Subunit-Vakzine isolierten Antigene können ebenfalls in hochgereinigter Form und in hoher Konzentration in der Vakzine enthalten sein. Jedoch kann die Immunogenität im Vergleich zu einer Gesamtvirus-Vakzine reduziert sein, da nur einzelne Antigene als Immunogen zur Verfügung stehen.

Um eine ausreichende Immunantwort bei der Vakzinierung zu induzieren, wurden daher sogenannte Split-Vakzine hergestellt. Dabei wird das Virus mit einem Detergens oder einer Mischung aus Detergens und einem Lösungsmittel vollständig solubilisiert, die Integrität des Virus zerstört und das Virus in seine einzelnen Komponenten, wie Kernproteine, Hüllproteine und Membranbestandteile aufgelöst. Das so hergestellte Gemisch aus viralen Komponenten wird dann für die Vakzinierung eingesetzt.

Versuche im Zuge der Virusinaktivierung von menschlichen Plasmaprodukten haben gezeigt, daß eine Lösungsmittel/Detergens-Behandlung mit TNBP/Tween® 80, in der Lage ist, lipidumhüllte Viren wie z.B. HIV, HCV, HBV oder Sindbisvirus zu inaktivieren (Piet et al., Transfusion 30 (1990), 591-598).

Bei der kommerziellen Produktion von Influenzavirus-Subunit-Vakzinen werden verschiedene Verfahren zur Inaktivierung mit Lipid-Lösungsmittel wie Triton® X-100, Cetyltrimethyl-Ammonium-Bromid, TNBP/Tween® 80 oder Diethylether/Tween® 80 eingesetzt. Insbesondere bei der Verwendung von Diethylether im Inaktivierungsschritt wurde festgestellt, daß ein markanter Abfall der Hämagglutinin-Aktivität erfolgt. Danihelkova et al. (Acta Virol. 28 (1984), 26-32) fanden, daß die Hämagglutinin- und Neuraminidase-Aktivität von inaktivierten Influenzavirus nach der Behandlung mit TNBP/Tween® 80 oder Diethylether/Tween® unter bestimmten Bedingungen erhalten bleibt. Sie merken jedoch an, daß die Entfernung des Lösungsmittels, insbesondere von Diethylether, aus der Präparation problematisch ist, und daher ein Inaktivierungsverfahren mit TNBP/Tween® 80 als bevorzugt angesehen wird.

Um die aufwendige Entfernung des Lösungsmittels zu vermeiden, wurde bei der Herstellung einer Influenza-Split-Vakzine lediglich Triton X-100 als nicht-ionisches Detergens verwendet (Gross et al., J. Clin. Microbiol. 14 (1981), 534-538).

Nicht-ionische Detergenzien besitzen keine geladenen Gruppen, der hydrophile Charakter dieser Detergenzien wird durch die Hydroxyl-Gruppe hervorgerufen. Im Gegensatz zu ionischen Detergenzien solubilisieren sie Membranproteine wesentlich milder. Das nicht-ionische Detergens löst Lipid-Lipid- und Lipid-Protein-Verbindungen, während Protein-Protein-Interaktionen unbeeinflußt bleiben. Dadurch wird die native Struktur der Proteine erhalten. Zudem ersetzt das Detergens die Lipide, die normalerweise mit dem hydrophoben Anteil der Proteine verbunden sind, wodurch sie eine lipid-ähnliche Umgebung schaffen und so solubilisierte Proteine stabilisieren können.

In der US 4 314 997 wird ein Verfahren zur Zerstörung von Endotoxinen und koagulierungsaktiven Substanzen sowie zur Inaktivierung von Hepatitis-Viren unter Verwendung von 0,25% bis 10% nicht-denaturierenden Amphiophilen beschrieben. In der EP 0 278 482 A2 wird ein Verfahren zur Inaktivierung hüllhaltiger Viren durch Behandeln mit einem nicht-ionischen Detergens erwähnt. Die US 4 673 733 A betrifft ein Verfahren zur Inaktivierung von Viren und Pyrogenen unter Verwendung eines Inaktivierungsgens. Die US 4 164 565 behandelt ein Vakzin gegen virale Hepatitis mit Hepatitis B-Oberflächenantigenen, einschließlich "e-Antigenen".

Untersuchungen zur Protektivität inaktivierter SIV-Vakzine nach Behandlung mit Formalin, Psoralem, Triton® X-100 oder Tween® /Ehter zeigten, daß Formalin- und Psoralen-inaktivierte SIV-Präparationen keinen Schutz gegen eine SIV-Infektion ergeben und die Triton® X-100-Inaktivierung nur zu einer teilweisen Protektion führt. Mit einer Tween® 80/Ether inaktivierten Split-Vakzine von SIV konnte jedoch erst bei einer hohen Antigendosis und in Gegenwart eines potenten Adjuvans eine Schutzwirkung gezeigt werden (Stahl-Hennig et al., Virology 186 (1992), 588-596).

Insbesondere bei der Verwendung von infektiösen humanpathogenen Viren zur Herstellung einer Ganzvirus-Vakzine ist es erforderlich, ein effektives und sicheres Verfahren zur Inaktivierung der Viren zur Verfügung zu stellen. Dies gilt insbesondere für solche Viren, wie etwa HIV, bei denen eine Inaktivierung mit herkömmlichen Methoden als für nicht ausreichend angesehen wurde. Wie oben schon erwähnt, führt bei SIV z.B. eine Formalin-Inaktivierung zu einem möglichen Verlust der Antigenität.

Ziel der vorliegenden Erfindung ist es daher, ein neues Verfahren zur Inaktivierung von Viren zur Verfügung zu stellen, das effizient lipidumhüllte Viren inaktiviert.

Die Aufgabe wird erfindungsgemäß dadurch gelöst, indem ein Verfahren zur Inaktivierung von lipidumhüllten Viren zur Verfügung gestellt wird, wobei ein lipidumhülltes intaktes Virus mit Verfahren zur Inaktivierung von lipidumhüllten Viren zur Verfügung gestellt wird, wobei ein lipidumhülltes intaktes Virus mit einem nicht-ionischen Detergens aus der Gruppe der Polysorbate inkubiert wird, über einen Zeitraum, der ausreicht, das Viruspartikel vollständig zu inaktivieren, ohne jedoch seine strukturelle Integrität und insbesondere die biologische Aktivität der Oberflächen- und Hüllproteine zu beeinflussen.

Überraschenderweise wurde gefunden, daß durch die Behandlung von lipidumhüllten Viren mit einem nicht-ionischen Detergens aus der Gruppe der Polysorbate die Viren effizient inaktiviert werden, wobei die strukturelle Integrität des Gesamtvirus und insbesondere der Hüllproteine, sowie deren biologische Aktivität, wie Immunogenität und Antigenität, zumindest zum überwiegenden Teil, erhalten bleibt. Dies war insbesondere deshalb überraschend, da die Verwendung von nicht-ionischen Detergenzien, wie Triton® X-100, im Stand der Technik zur Herstellung von Split-Vakzinen und damit zur Desintegration von intakten Viruspartikeln beschrieben ist.

Im Rahmen der Erfindung durchgeführte Vergleichsversuche mit anderen nicht-ionischen Detergenzien, wie etwa Octyl-Glucosid, haben zudem gezeigt, daß dieses Detergens zwar alle getesteten Viren inaktiviert, die strukturellen Proteine des Virus, insbesondere die Hüllproteine, jedoch angreift und solubilisiert. Da für die Ausbildung einer protektiven Immunantwort von viralen Vakzinen die Erhaltung der biologischen Aktivität, insbesondere die äußeren Hüllproteine, von entscheidender Bedeutung ist, ist die Antigenität und Immunogenität einer Vakzine, die ein Gesamtvirus mit solubilisierten Hüllproteinen enthält, im Vergleich zu einem intakten Virus beeinträchtigt.

Gemäß einem besonderen Aspekt der Erfindung werden bei der Durchführung des erfindungsgemäßen Verfahrens lipidumhüllte Viren inaktiviert. Besonders bevorzugt sind dabei lipidumhüllte Viren aus der Gruppe der Retroviren, wie HIV-1 und HIV-2, der Flaviviren, wie TBEV und HCV, der Influenzaviren, der Herpesviren, der Paramyxoviren, wie Mumpsvirus und Masernvirus, oder der Arenaviren, wie Juninvirus und Lassavirus.

Es hat sich zudem gezeigt, daß durch das erfindungsgemäße Verfahren nicht nur die Immunogenität der Antigene und die Integrität des Gesamtvirus und der Hüllproteine erhalten bleibt, sondern daß die biologische Aktivität der Proteine, wie etwa die hämagglutinierende Aktivität bei Influenzavirus oder TBEV oder die CD4-Bindungsaktivität bei HIV-1 nicht beeinflußt wird.

Zur Durchführung des erfindungsgemäßen Verfahrens werden nicht-ionische Detergenzien aus der Gruppe der Polysorbate (Polyoxyethylensorbitane) eingesetzt. Vorzugsweise werden dabei Polysorbate aus der Gruppe von Tween® 80, Tween® 60, Tween® 40 und Tween® 20 oder Kombinationen derselben verwendet. Besonders bevorzugt bei der Durchführung des Verfahrens ist die Verwendung von Tween® 80. Es hat sich insbesondere gezeigt, daß die nicht-ionischen Detergenzien dieser Gruppe intakte Proteine nicht angreifen bzw. ihre Aktivität nicht zerstören.

Insbesondere zur vollständigen Inaktivierung aller Viren in einer Lösung ist erforderlich, daß eine Interaktion und Aggregatbildung der Viruspartikel vermieden wird, da innerhalb eines aggregierten Komplexes einzelne Partikel gegen die Einwirkung des inaktivierenden Detergens geschützt sein können. Das vorliegende Verfahren bietet daher den Vorteil, daß die Interaktion von Viren über Protein-Protein-Wechselwirkung der Oberflächenproteine reduziert und sogar verhindert wird und die einzelnen vorliegenden Viruspartikel direkt vom Detergens umhüllt und inaktiviert werden können.

Zur Durchführung des erfindungsgemäßen Verfahrens wird eine Konzentration des Polysorbates zur Inaktivierung der lipidumhüllten Viren in einem Bereich zwischen 1% und 25%, insbesondere zwischen 1% und 20%, eingesetzt. Vorzugsweise liegt die Konzentration zwischen 10% und 20% bzw. zwischen 10% und 15%.

Es wurde im Rahmen der Erfindung überraschenderweise festgestellt, daß eine vollständige Inaktivierung der getesteten Viren schon nach 10 min Inkubation mit dem Polysorbat erfolgte, während in einem parallelen Versuchansatz mit 0,03 bis 0,1% Formalin eine vergleichbare Inaktivierung von z.B. HIV-1 erst nach 10 Stunden erreicht wurde.

Das vorliegende Verfahren zur Inaktivierung von lipidumhüllten Viren wird daher erfindungsgemäß derart durchgeführt, daß eine Virussuspension, die entweder direkt von Zellkulturüberständen stammt oder aus einer gereinigten Viruslösung besteht, mit dem nicht-ionischen Detergens aus der Gruppe der Polysorbate für mindestens 10 min inkubiert wird. Bei den meisten lipidumhüllten Viren wird schon nach diesem Zeitraum eine komplette Inaktivierung der Viren in der Lösung erreicht. Längere Inkubationszeiten bis zu 10 Stunden sind jedoch ohne Verlust der biologischen Aktivität und der strukturellen Integrität des Gesamtvirus und der Hüllproteine möglich.

Der Zeitraum der Inkubation mit dem nicht-ionischen Detergens, der ausreicht, um das Virus zu inaktivieren, kann dabei durch eine einfache Kinetik der Virusinaktivierung und Bestimmung des Virustiters, wie etwa in Beispiel 2 (siehe unten) beschrieben, ermittelt werden.

Bevorzugterweise wird das erfindungsgemäße Verfahren bei einer Temperatur zwischen 20°C und 40°C durchgeführt. Die Auswahl des optimalen Temperaturbereichs zur Inaktivierung von Viren mit dem erfindungsgemäßen Verfahren gehört dabei zum allgemeinen Wissen eines Fachmannes. Die Ermittlung einer optimalen Temperatur für das erfindungsgemäße Verfahren kann daher ohne Schwierigkeit für die jeweilige Kombination Detergen/Virus durchgeführt werden. Ebenso können weitere Parameter, wie die optimale Konzentration des nicht-ionischen Detergens, die Proteinkonzentration und Inkubationsdauer von jedem Fachmann ermittelt werden.

Bei der Ermittlung der optimalen Verfahrensparameter ist stets darauf zu achten, die strukturelle Integrität und insbesondere die biologische Aktivität der Oberflächen- und Hüllproteine nicht zu beeinflussen. Als ausreichend ist hiefür zwar eine zumindest 50%-ige Erhaltung der ursprünglich enthaltenen Ganzviren anzusehen, bevorzugterweise sollten jedoch mindestens 80%, noch bevorzugter mindestens 90%, insbesondere mindestens 95%, der erfindungsgemäß behandelten Viruspartikel ihre strukturelle Integrität beibehalten.

Ein besonderer Vorteil des erfindungsgemäßen Verfahrens liegt damit bei der schnellen und effizienten Inaktivierung der lipidumhüllten Viren, die insbesondere die Einsetzbarkeit des Verfahrens im großtechnischen Maßstab und bei der Produktion von inaktivierten Virus-Vakzinen ermöglichen. Da bekanntermaßen für die Herstellung z. B. einer Influenzavirus-Vakzine nur eine kurze Periode für die Virusvermehrung, Inaktivierung und Impfstoffzubereitung zur Verfügung stehen, bietet das vorliegende Verfahren in der industriellen Vakzineproduktion eine wesentliche Verbesserung.

Im Anschluß an die Virusinaktivierung erfolgt bei der Produktion von Vakzinen in jedem Fall eine Sterilfiltration, um etwaige Kontaminationen, wie zelluläre Bestandteile oder bakterielle Verunreinigungen, aus der Lösung zu entfernen.

Im Rahmen der vorliegenden Erfindung wurde festgestellt, daß eine durch das vorliegende Verfahren hergestellte inaktivierte Viruspräparation einfacher zu sterilfiltrieren ist, da die Glykoproteine der äußeren Virushülle, die normalerweise an die Filtermembran binden können, durch die Bindung mit den Detergensmonomeren eine geringere Affinität zur Filtermembran aufweisen. Damit bietet das vorliegende Verfahren den zusätzlichen Vorteil, daß die bei der Sterilfiltration normalerweise auftretenden Verluste an inaktivierten Viruspartikel reduziert werden und die Ausbeute an inaktivierten Viren für die Vakzinherstellung erhöht wird.

Gemäß einem besonderen Aspekt der vorliegenden Erfindung verbleibt das nicht-ionische Detergens aus der Gruppe der Polysorbate nach dem Inaktivierungsschritt in der virushaltigen Lösung. Insbesondere für Tween® 80 ist bekannt, daß es als humanverträglich gilt und vielfach in Lebensmitteln und Kosmetika Verwendung findet. Es sind daher bei einer am Menschen oder Tier zu verabreichenden Vakzine, enthaltend eine mehr oder weniger geringe Menge an Detergens keine Nebenwirkungen zu erwarten. Da gemäß dem vorliegenden Verfahren für die Inaktivierung von Viren eine Konzentration von bis zu 20% an nicht-ionischem Detergens eingesetzt werden kann, ist es jedoch in manchen Fällen vorteilhaft, die Konzentration an Detergens im Endprodukt zu reduzieren oder gegebenenfalls ganz zu entfernen. Dies kann durch bekannte Maßnahmen, wie etwa Dialyse oder chromatographische Verfahren, erfolgen. Besonders geeignete chromatographische Verfahren sind dabei Ionen-Austauschchromatographie oder Gelfiltration. Es eignen sich jedoch alle im Stand der Technik beschriebenen Verfahren zur Entfernung von nicht-ionischen Detergenzien aus einer Lösung.

Bei der Verwendung von viralen Impfstoffen, die insbesondere Oral verabreicht werden, besteht oft das Problem, daß die Antigene durch proteolytischen Abbau im Magen zerstört werden und somit ihre Wirkung einbüßen. Polysorbate gelten bekanntermaßen auch als "stabilisierende Detergenzien", die in einer Lösung vorhandenen Komponenten, insbesondere Proteine, erlauben, über einen längeren Zeitraum in der Lösung stabil zu bleiben (WO 93/01831). Gemäße einem weiteren Aspekt der vorliegenden Erfindung enthält daher die erfindungsgemäß hergestellte inaktivierte Viruspräparation ein stabilisierendes Agens. Vorzugsweise enthält die Viruspräparation als stabilisierendes Agens ein Polysorbat in einer Endkonzentration von mindestens 0,05%, vorzugsweise von 0,05% bis 0,5%, besonders bevorzugt von 0,2%. Es ist jedoch auch möglich, daß als Inaktivierungsmittel verwendete Detergens aus der Viruspräparation zu entfernen und jedes beliebige aus dem Stand der Technik bekannte stabilisierende Agens der Präparation zuzusetzen.

Ein weiterer Aspekt der Erfindung betrifft ein Verfahren zur Herstellung einer inaktivierten Virus-Vakzine, wobei nach der Inaktivierung gemäß dem oben beschriebenen Verfahren erhaltene inaktivierte Viren, gegebenenfalls nach Entfernen des inaktivierenden Detergens, mit einem physiologisch akzeptablem Träger und gegebenenfalls mit einem Adjuvans versetzt werden.

In der mit dem erfindungsgemäßen Verfahren erhaltenen Viruspräparation enthaltend ein inaktiviertes Virus, zeichnet sich das Virus durch die strukturelle Integrität des Gesamtvirus und insbesondere der Integrität der Hüllproteine aus. Es wurde im Rahmen der vorliegenden Erfindung festgestellt, daß die Behandlung und Inaktivierung eines lipidumhüllten Viruspartikels mit einem nicht-ionischen Detergens, ausgewählt aus der Gruppe der Polysorbate, die Integrität des Gesamtvirus nicht beeinträchtigt und insbesondere die biologische Aktivität der Hüllglykoproteine nicht beeinflußt. Dies war insbesondere deshalb überraschend, da andere nicht-ionische Detergenzien, wie Triton X-100 oder Octyl-Glucoside lipidumhüllte Viren inaktivieren, und dabei die strukturelle Integrität des gesamten Virus zerstören.

Die erfindungsgemäß erhaltene Viruspräparation kann gegebenenfalls als weiteren Bestandteil ein stabilisierendes Agens enthalten. Entsprechend den obigen Ausführungen ist das stabilisierende Agens vorzugsweise das zur Inaktivierung verwendete nicht-ionische Detergens aus der Gruppe der Polysorbate, insbesondere Tween® . Es kann jedoch jedes aus dem Stand der Technik bekannte stabilisierende Agens enthalten sein.

Die erfindungsgemäß erhaltene Viruspräparation kann als Arzneimittel, insbesondere - gemäß einem weiteren Aspekt der Erfindung - zur Herstellung eines stabilen Impfstoffes zur Immunisierung von Vertebraten, also einen Impfstoff enthalten eine Viruspräparation der oben beschriebenen Art und gegebenenfalls enthaltend einen physiologisch akzeptablen Träger zur Verfügung gestellt werden.

Dabei besteht der Impfstoff aus einer nach dem erfindungsgemäßen Verfahren inaktivierten Viruspräparation, wobei gegebenenfalls nach dem Inaktivierungsschritt das inaktivierende nicht-ionische Detergens entfernt und ein physiologisch akzeptabler Träger der inaktivierten Viruslösung zugesetzt wird. Als physiologisch akzeptable Träger kommen gemäß der vorliegenden Erfindung alle im Stand der Technik bekannten pharmazeutischen Formulierungen in Betracht. Die Formulierung des erfindungsgemäßen Impfstoffes kann in an sich bekannter und üblicher Weise erfolgen, z.B. mit Hilfe von ionisiertem Wasser, von Phosphat-gepufferter Lösung, von Salzen, und gegebenenfalls zur Stabilisierung Aminosäuren oder milde nicht-ionische Detergenzien, wie z.B. Tween® 20 oder Tween® 80, enthalten.

Das inaktivierte Virus kann auf die verschiedensten Arten für den Gebrauch als Vakzine formuliert sein. Die Konzentration des inaktivierten Virus im Impfstoff liegt im allgemeinen zwischen 10⁶ und 10⁹ pfu/ml, vorzugsweise bei 10⁷ pfu/ml. Der erfindungsgemäße Impfstoff enthält gegebenenfalls zur Erhöhung der Immunantwort, z.B. zur Stimulierung der Produktion von neutralisierenden Antikörpern, ein Adjuvans in einer gewünschten Konzentration. Immunologisch akzeptable Adjuvantien können dabei Mineralöle, Freund'sches Adjuvans, pflanzliche Öle, Mineralsalze, Immunmodulatoren oder Immunpotentiatoren sein.

Der Impfstoff kann auf verschiedenste Weise, z.B. subkutan, oral, nasal, intramuskulär oder intraperitonal verabreicht werden. Im allgemeinen wird die Impfstoffdosis nach bekannten Impfschemen verabreicht.

Die Erfindung wird an Hand der nachfolgenden Beispiele und der Zeichnungsfiguren näher beschrieben, wobei sie jedoch selbstverständlich nicht auf diese eingeschränkt sein soll.

Beispiel 1 beschreibt die Inaktivierung lipidumhüllter Viren mit nicht-ionischen Detergenzien Tween® 80 und Octyl-Glucosid; Beispiel 2 beschreibt die Kinetik der Virusinaktivierung von HIV-1 und TBEV nach Tween® 80-Behandlung; Beispiel 3 beschreibt die Westernblot-Analyse von inaktiviertem HIV-1 nach Behandlung mit 1% Octyl-Glucosid und 15% Tween® 80; Beispiel 4 beschreibt die Westernblot-Analyse von inaktiviertem KP-Influenzavirus nach Behandlung mit 1% Octyl-Glucosid und 11% Tween® 80; Beispiel 5 beschreibt die Immunisierung von Mäusen mit Tween® 80-inaktivierten MAIDS-Komplex; Beispiel 6 beschreibt die Kinetik der Virusinaktivierung von HIV-1 mit Formalin und Beispiel 7 beschreibt die Westernblot-Analyse von Formalin-inaktiviertem HIV-1 nach verschiedener Inkubationsdauer.
- Figur 1:: Westernblot-Analyse von inaktiviertem HIV-1 nach Behandlung mit 1% Octyl-Glucosid und 11% Tween® 80
- Figur 2:: Westernblot-Analyse von inaktiviertem KP-Influenzavirus nach Behandlung mit 1% Octyl-Glucosid und 15% Tween® 80
- Figur 3:: Westernblot-Analyse von Formalin-inaktiviertem HIV-1 nach verschiedener Inkubationsdauer
- Tabelle 1:: Virusinaktivierung lipidumhüllter Viren mit nichtionischen Detergenzien Tween® 80 und Octyl-Glucosid
- Tabelle 2:: Kinetik der Virusinaktivierung von HIV-1 und TBEV nach Tween® 80-Behandlung
- Tabelle 3:: Immunantwort von mit Tween® 80-inaktiviertem MAIDS-Komplex immunisierten Mäusen nach 5 Wochen und 12 Wochen
- Tabelle 4:: Kinetik der Virusinaktivierung von HIV-1 mit Formalin

### Beispiel 1:

Virusinaktivierung lipidumhüllter Viren mit nicht-ionischen Detergenzien Tween® 80 und Octyl-Glucosid

Viruspräparationen von HIV-1, HIV-2, Influenzavirus und PRV (Pseudorabiesvirus) wurden über Saccharose-Gradienten-Zentrifugation und anschließende Dialyse gereinigt. Die gereinigte Präparation wurde mit Octyl-Glucosid bis zu einer Endkonzentration von 1% oder Tween® 80 bis zu einer Endkonzentration von 11%, 15% und 20% versetzt und für 1 Stunde bei 26°C inkubiert. Die Präparation wurde anschließend mit PBS verdünnt, für 5 Minuten bei 400,000 g pelletiert und vor der Titration in PBS resuspendiert. Derart behandelte HIV-1, HIV-2, Influenzavirus oder PRV wurden in halblogarithmischen Stufen in Zellkulturmedium verdünnt. Je 8 x 100 µl jeder Verdünnungsstufe wurden in eine Vertiefung einer Mikrotiterplatte, enthaltend Indikatorzellen, zugegeben. Die infizierten Zellen wurde bei 37°C für 5 bis 6 Tage inkubiert und der cytopathische Effekt des Virus auf die Indikatorzelle mikroskopisch bestimmt. Als Indikatorzellen wurden für PRV und Influenzavirus Vero-Zellen, für HIV-1 AA2-Zellen und für HIV-2 C8166-Zellen verwendet. Die TCID₅₀ wurde gemäß Reed und Muench (Am. J. Hyg. 27 (1938), 493-497) ermittelt und ist in Tabelle 1 zusammengefaßt.

Die Daten der Tabelle 1 zeigen, daß mit 1% Octyl-Glucosid alle getesten Viren um 5-7 log₁₀ inaktiviert wurden. Ebenfalls alle Viren wurden mit 20% Tween® 80 inaktiviert, während für die Inaktivierung von HIV-1, HIV-2 und TBEV schon eine Konzentration von 11% Tween® 80 für eine komplette Inaktivierung ausreichte.

**Tabelle 1**

| Inaktivierung lipidumhüllter Viren mit den nichtionischen Detergenzien Twenn® 80 une Octyl-Glucosid | | | | | |
|---|---|---|---|---|---|
| Virus | Virus Titer (log₁₀ TCID₅₀/ml) | | | | |
| | Behandlung | | | | |
| | PBS | 1% Octyl-Glucoside | 11% Tween-80 | 15% Tween-80 | 20% Tween-80 |
| HIV-1 | 6.7 | ≤ 0.5 | ≤0.5 | ≤0.5 | ≤0.5 |
| HIV-2 | 6.1 | ≤0.5 | ≤0.5 | ≤0.5 | ≤0.5 |
| TBEV | 7.0 | ≤0.5 | ≤0.5 | ≤0.5 | ≤0.5 |
| Influenza | 7.4 | ≤0.5 | 2.9 | ≤0.5 | ≤0.5 |
| PRV | 7.2 | 1.1 | 2.5 | 1.7 | ≤0.5 |

### Beispiel 2:

### Kinetik der Virusinaktivierung von HIV-1 und TBEV nach Tween® 80-Behandlung

Eine Saccharose-Gradienten gereinigte Viruspräparation von TBEV und HIV-1 wurde mit einer Tween® 80-Lösung bis zu einer Endkonzentration von 11% Tween® 80 gemischt. Die Mischung wurde bei 26°C unter ständigem Rühren inkubiert und nach verschiedenen Zeiten Proben zur Virustitration entnommen. Die Proben wurden sofort nach Entnahme im Verhältnis 1:20 mit PBS verdünnt, bei 400,000 g für 5 Minuten pelletiert und das Pellet vor der Titration in PBS resuspendiert. TBEV wurde über einen Plaque-Assay auf PS-Zellen und HIV-1 auf AA2-Zellen titriert.

Das TBE-Virus wurde stufenweise in log 1-Schritten verdünnt und je zwei Aliquote von 500 *µ*l jeder Verdünnungsstufe zu Zellmonolayern gegeben. Die Zellen wurden mit einer 1:1-Mischung von 2 x Medium und 1,8% Carboxymethylcellulose überschichtet und für 4 Tage inkubiert. Virale Plaques wurden durch Färbung mit 0,1% Kristallviolett-Lösung visualisiert und der Titer durch Auszählen der Plaques bestimmt.

Die Titration von HIV-1 auf AA2-Zellen erfolgte nach stufenweiser Verdünnung in Zellkulturmedium. Je 8 x 100 *µ*l jeder Verdünnungsstufe wurden in die Vertiefungen einer Mikrotiterplatte enthaltend 2 x 10⁴ AA2-Zellen gegeben. Die infizierten Zellen wurden bei 37°C für 5-6 Tage inkubiert und der cytopathische Effekt des Virus auf die Indikatorzellen mikroskopisch bestimmt. Die TCID₅₀ wurde gemäß Reed und Muench (Am. J. Hyg. 27 (1938), 493-497) ermittelt.

Der Titer (pfu/ml) wurde entsprechend der Poisson-Verteilung ermittelt. Die Ergebnisse sind in Tabelle 2 zusammengefaßt.

Die Daten der Tabelle 2 zeigen, daß durch 11% Tween® 80 eine TBEV-Präparation innerhalb von 20 Minuten bei 26°C um >6,4 log₁₀ inaktiviert wird. Unter den gleichen Bedingungen wird eine HIV-Präparation innerhalb von 10 Minuten um >6,2 log₁₀ inaktiviert.

**Tabelle 2:**

| Kinetik der Virusinaktivierung von HIV-1 und TBEV nach Tween® 80-Behandlung | | |
|---|---|---|
| Behandlungsdauer (min) | TBEV Titer (PFU/ml) | HIV-1 Titer (TCID₅₀/ml) |
| 0 | 10^{7.4} | 10^{6.7} |
| 5 | 10^{3.9} | n.b. |
| 10 | 10^{1.5} | < 10^{0.5} |
| 20 | < 10^{1.0} | <10^{0.5} |
| 40 | < 10^{1.0} | < 10^{0.5} |
| 60 | < 10^{1.0} | < 10^{0.5} |

### Beispiel 3:

### Westernblot-Analyse von inaktiviertem HIV-1 nach Behandlung mit 1% Octyl-Glucosid und 11% Tween® 80

Eine Saccharose-Gradienten gereinigte Präparation von HIV-1 wurde gemäß Beispiel 1 jeweils mit 11% Tween® 80, 1% Octyl-Glucosid oder PBS gemischt und bei 26°C für 1 Stunde inkubiert. Die Proben wurden anschließend verdünnt, für 10 Minuten bei 4°C mit 400,000 g pelletiert (wobei Virusproteine, insbesondere Hüllproteine, die durch die Detergens-Behandlung vom Ganzvirus gelöst werden,wobei das Virusteilchen seine strukturelle Integrität verliert, im Überstand bleiben und daher von der nachfolgenden Westernblot-Analyse nicht mehr erfaßt werden), in Lysepuffer resuspendiert, mittels SDS-PAGE nach Laemmli (Nature 227 (1970), 680-685) aufgetrennt und auf eine Filtermembran geblottet.

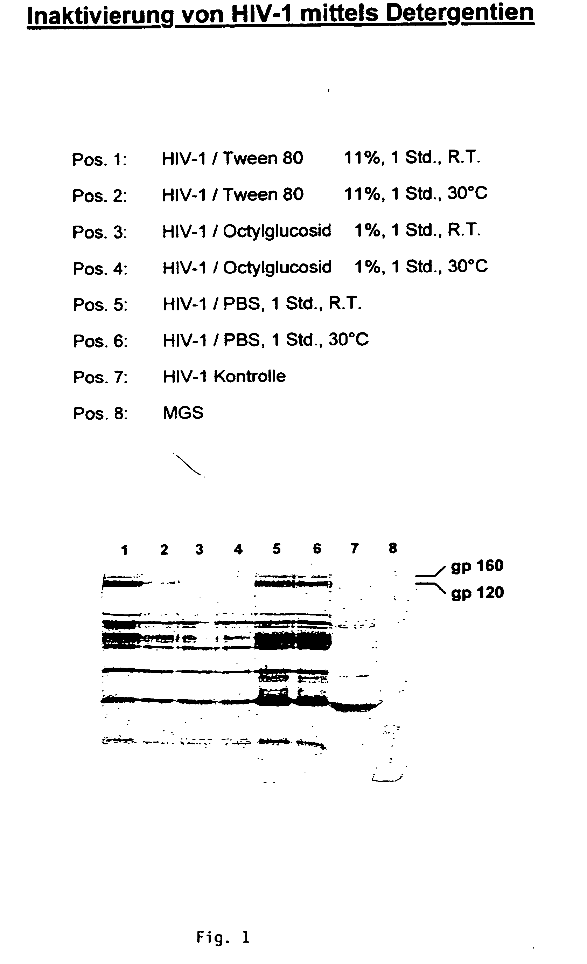

Zur Detektion HIV-1-spezifischer Proteine wurde die Filtermembran mit einem Gemisch aus HIV-1-Antikörper-positivem Human-IgG (Immuno AG), einem gp41-spezifischen humanen monoklonalen Antikörper und einem gp120-spezifischen monoklonalen Maus-Antikörper (DuPont) über Nacht inkubiert. Nach dem Waschen wurde die Membran mit Peroxidase-gekoppelten anti-Human- und anti-Maus-Ziegen-IgG als zweitem Antikörper inkubiert. Der Nachweis HIV-1-spezifischer Banden erfolgte über eine enzymatische Farbreaktion mit Diaminobenzidin und H₂O₂. Das Ergebnis der Westernblot-Analyse ist in Figur 1 gezeigt.

Nach der Behandlung mit 1% Octyl-Glucosid können die HIV-1-Hüllproteine, insbesondere gp160 und gp120, nicht mehr nachgewiesen werden, was belegt, daß die strukturelle Integrität der Octyl-Glucosid-behandelten Viruspartikel bzw. die biologische Aktivität der Oberflächen- oder Hüllproteine verlorengegangen ist. Im Gegensatz dazu können sowohl bei der Kontrolle (PBS) als auch bei der Tween® 80-behandelten Probe deutlich die Hüllproteine im Westernblot detektiert werden, was beweist, daß die strukturelle Integrität des gesamten Virus bei der Tween-Behandlung erhalten bleibt. Durch die Immun-Reaktion mit dem gp120-spezifischen Antikörper wird ebenfalls gezeigt, daß die antigenen Determinanten der Hüllproteine durch die Tween® 80-Behandlung nicht beeinflußt werden und keine Veränderungen im Vergleich zur nichtbehandelten Kontrolle aufweisen.

### Beispiel 4:

### Westernblot-Analyse von inaktiviertem KP-Influenzavirus nach Behandlung mit 1% Octyl-Glucosid und 15% Tween 80

Eine Saccharose-Gradienten gereinigte Präparation von KP-Influenzavirus wurde entsprechend Beispiel 1 und 3 mit 1% Octyl-Glucosid, 15% Tween 80 oder PBS behandelt, pelletiert und eine Westernblot-Analyse durchgeführt.

Zur Detektion von Influenzavirus-spezifischen Proteinen wurde die Filtermembran mit monoklonalen Maus-Antikörpern, gerichtet gegen HA1 und HA2 des Oberflächenglykoproteins, Hämagglutinin (HA), über Nacht inkubiert. Nach dem Waschen wurde die Membran mit Peroxidase-gekoppelten anti-Human- und anti-Maus-Ziegen-IgG als zweitem Antikörper inkubiert. Der Nachweis der Influenzavirus HA-Glykoproteine erfolgte über eine enzymatische Farbreaktion mit Diaminobenzidin und H₂O₂.

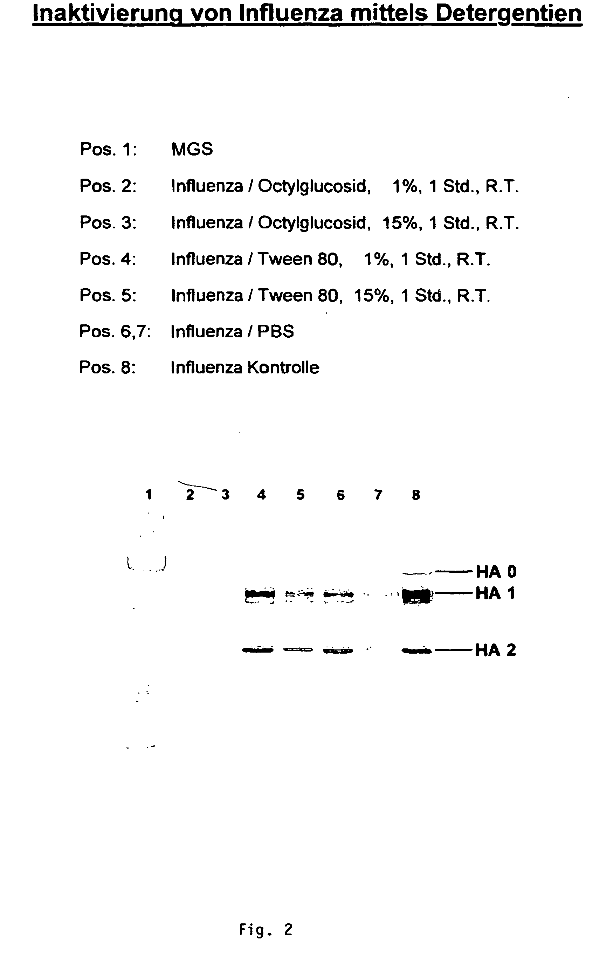

Die Ergebnisse der Westernblot-Analyse sind in Figur 2 dargestellt.

Durch die Behandlung mit 1% Octyl-Glucosid werden die HA-Glykoproteine von Influenzavirus vollständig entfernt (Figur 1; Spalte 2 und 3). Im Gegensatz dazu, ist sowohl bei der Kontrolle (PBS) als auch bei der Tween® 80-behandelten Probe deutlich die strukturelle Integrität der HA-Glykoproteine zu erkennen (Figur 2, Spalte 4 bis 7).

### Beispiel 5:

### Immunisierung von Mäusen mit Tween® 80-inaktivierten MAIDS-Komplex

Ein MAIDS-Komplex (BM5-Mix MuLV)-Präparation wurde 1 Stunde mit Tween® 80 oder mit PBS bei Raumtemperatur inkubiert. MAIDS-Komplex-sensitiven C57 B1/6 wurde i.p. eine Tween® 80-inaktivierte und eine PBS-behandelte MAIDS-Komplex-Präparation injiziert. 5 und 12 Wochen nach der Immunisierung wurden die Mäuse seziert. Zur Beurteilung der MAIDS-Symptome wurde das Gewicht der cervicalen Lymphknoten und der Milzen bestimmt, infektiöse Viren aus der Milz isoliert und der Antikörper-Titer gegen die Antigene des BM5-Mix MuLV-Komplexes bestimmt. Die Ergebnisse sind in Tabelle 3a und 3b zusammengefaßt.

Tabelle 3a und 3b zeigen, daß die Behandlung des BM5-Mix MuLV-Komplexes mit Tween® 80 zu einer vollständigen Inaktivierung führte. Es traten über den Beobachtungszeitraum von 12 Wochen keine MAIDS-Symptome auf und es konnte kein infektiöses Virus aus den Milzzellen isoliert werden. Die inaktivierte Viruspräparation induzierte aber relativ hohe Antikörper-Titer gegen die Antigene des MAIDS-Komplexes. Die mit PBS behandelte BM5-Mix MuLV-Präparation führte zu eindeutigen MAIDS-Symptomen mit einer großen Zunahme des Milzgewichtes und der cervicalen Lymphknoten, sehr hohen Virustitern in der Milz und, bedingt durch die MAIDS-Komplex induzierte Immunschwäche, zu keiner Immunantwort.

**Tabelle 3a :**

| **MAIDS / Ergebnisse Tween-Inaktivierung** | | | | | | | | |
|---|---|---|---|---|---|---|---|---|
| Sektions-Ergebnisse, Elisa - Titer und Isolierung des ecotropen MuLV aus den Milzen infizierter C57 BI/6 - Mäuse 5 Wochen nach der Infektion mit BM5-Mix MuLV | | | | | | | | |
| Gruppe | Infektion | Tier | Sektion | | | | Elisa - Titer | log 10 Titer infizierte Milzzellen / 7,0 Milzzellen |
| | | | Vergrößerung | | Gewicht | | | |
| | | | Lymphknoten | Milz | Milz | cervicale Lymphknoten | BM5 Mix MuLV-Antigen | |
| A | BM5 Mix MuLV + 11% Tween 80 | 1 | - | - | 70mg | o.B. | 320 | 0 |
| | | 2 | - | - | 60mg | o.B. | 160 | |
| | | 3 | - | - | 70mg | o.B. | 80 | |
| | | 4 | - | - | 60mg | o.B, | 80 | |
| | | 5 | .. | - | 70mg | o.B. | 160 | |
| B | BM5 Mix MuLV Puffer | 1 | + | + | 170mg | 30mg | < | 3,3 |
| | | 2 | + | + | 180mg | 90mg | < | |
| | | 3 | + | +/++ | 260mg | 70mg | < | |
| | | 4 | + | + | 230mg | 80mg | < | |
| | | 5 | + | + | 220mg | 80mg | < | |

| | | | | | | | | |
|---|---|---|---|---|---|---|---|---|
| 0.B. = ohne Besonderheiten (Gewicht nicht bestimmbar) < = < 1:10 | | | | | | | | |

**Tabelle 3b:**

| **MAIDS / Ergebnisse Tween-Inaktivierung** | | | | | | | | |
|---|---|---|---|---|---|---|---|---|
| Sektions-Ergebnisse, Elisa - Titer und Isolierung des ecotropen MuLV aus den Miizen infizierter C57 BI/6 - Mäuse 12 Wochen nach der Infektion mit BM5-Mix MuLV | | | | | | | | |
| Gruppe | Infektion | Tier | Sektion | | | | Elisa - Titer | log 10 Titer infizierte Milzzellen / 7,0 Milzzellen |
| | | | Vergrößerung | | Gewicht | | | |
| | | | Lymphknoten | Milz | Milz | cervicale Lymphknoten | BM5 Mix MuLV·Antigen | |
| A | BM5 Mix MuLV + 11% Tween 80 | 6 | - | - | 80mg | o.B. | 80 | 0 |
| | | 7 | - | - | 90mg | o.B. | 80 | |
| | | 8 | - | - | 70mg | o.B. | 40 | |
| | | 9 | - | - | 70mg | o.B. | 40 | |
| | | 10 | - | - | 90mg | o.B. | 80 | |
| B | BM5 Mix MuLV + Puffer | 6 | +++ | ++ | 630mg | 610mg | < | 5,1 |
| | | 7 | +++ | ++/+++ | 710mg | 660mg | < | |
| | | 8 | +++/++++ | +++ | 1130mg | 1190mg | < | |
| | | 9 | +++ | ++ | 680mg | 680mg | < | |
| | | 10 | ++++ | +++ | 990mg | 1360mg | < | |

| | | | | | | | | |
|---|---|---|---|---|---|---|---|---|
| o.B. = ohne Besonderheiten (Gewicht nicht bestimmbar) < = < 1:10 | | | | | | | | |

### Beispiel 6:

### Kinetik der Virusinaktivierung von HIV-1 mit Formalin

Zu einer Saccharose-Gradienten-gereinigten Präparation von HIV-1 mit einem Virustiter von 10⁷ TCID_{50/}ml wurde Formalin mit einer Endkonzentration von 0,01%, 0,03%, 0,05%, 0,075% und 0,1% zugegeben. Die Lösung wurde bei 37°C unter ständigem Rühren inkubiert und nach unterschiedlichen Zeiten wurden Proben für die Virustitration entnommen. Jeder der Proben wurde vor der Titration zur Neutralisation von Formalin ein entsprechendes Aliquot an Na₂S₂O₅ zugesetzt. Die Virustitration erfolgte wie in Beispiel 1 beschrieben.

Bei einer Formalinkonzentration von 0,03% bis 0,1% erfolgte eine effiziente Inaktivierung von HIV-1 nach etwa 10 Stunden. Mit einer Konzentration unterhalb von 0,03% Formalin konnten nach 30-stündiger Inkubation noch aktive, infektiöse Viren bestimmt werden.

**Tabelle 4:**

| Kinetik der Virusinaktivierung von HIV-1 mit Formalin | | | | | | | |
|---|---|---|---|---|---|---|---|
| Formalin (%) | Zeit (h) | | | | | | |
| | 0 | 1 | 3 | 6 | 10 | 20 | 30 |
| 0.01 | 6.0 | 5.9 | 4.9 | 4.1 | 2.9 | 1.0 | 1.0 |
| 0.03 | 5.8 | 3.9 | 2.9 | 1.5 | ≤ 0.5 | ≤ 0.5 | ≤ 0.5 |
| 0.05 | 6.3 | 4.6 | 2.4 | n.b. | ≤ 0.5 | n.b. | ≤ 0.5 |
| 0.075 | 6.0 | 4.3 | 1.9 | n.b. | ≤ 0.5 | n.b. | ≤ 0.5 |
| 0.10 | 6.4 | 3.7 | 1.6 | n.b. | ≤ 0.5 | n.b. | ≤ 0.5 |

| | | | | | | | |
|---|---|---|---|---|---|---|---|
| n.b. = nicht bestimmt | | | | | | | |

### Beispiel 7:

### Westernblot-Analyse von Formalin-inaktiviertem HIV-1 nach verschiedener Inkubationsdauer.

Aus einer gemäß Beispiel 6 Formalin-inaktivierten HIV-1-Viruspräparation wurden nach 0, 1, 2, 3, 6, 10, 20 und 30 Stunden Proben entnommen und mit Aliquoten eine Westernblot-Analyse durchgeführt.

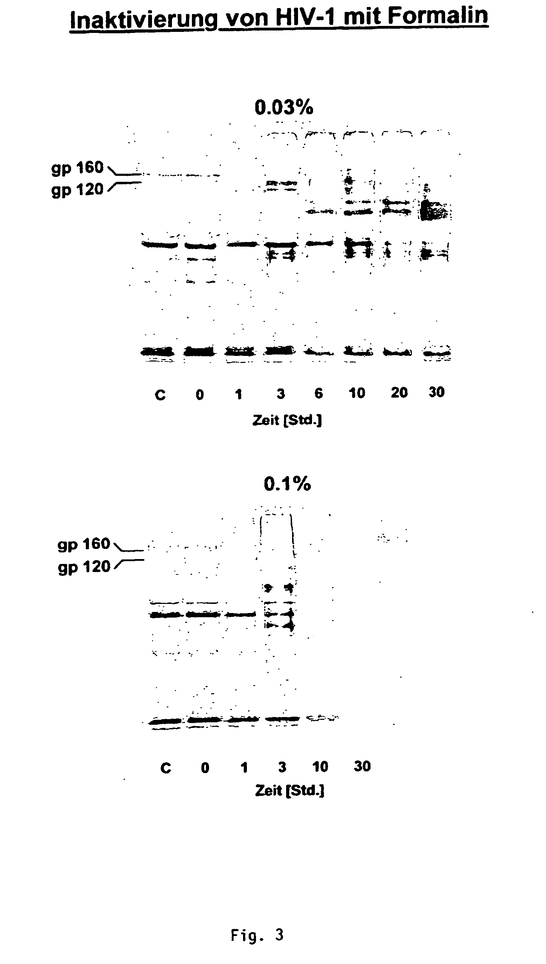

Zur Detektion HIV-1-spezifischer Proteine wurde die Filtermembran mit HIV-1-Antikörper-positivem Human-IgG (Immuno AG) über Nacht inkubiert. Nach dem Waschen wurde die Membran mit Peroxidase-gekoppelten anti-Human- und anti-Maus-Ziegen-IgG als zweitem Antikörper inkubiert. Der Nachweis HIV-1-spezifischer Banden erfolgte über eine enzymatische Farbreaktion mit Diaminobenzidin und H₂O₂. Das Ergebnis der Westernblot-Analyse ist in Figur 3 dargestellt.

Es zeigte sich, daß nach einer Formalin-Behandlung die Viren zwar pelletierbar und damit noch als Gesamtvirus intakt sind, jedoch nach bereits 1-stündiger Inkubationszeit mit 0,1% Formalin die Hüllproteine abgebaut werden, wobei die native Struktur der Proteine zerstört wird.

Die Westernblot-Analyse zeigt, daß ab 3 Stunden Inkubationsdauer eine Desintegration der intakten Viren erfolgte. Eine 10-stündige Inkubationsdauer, die gemäß Beispiel 6 für eine effiziente Inaktivierung der Viren notwendig ist, führt ebenfalls zu einer Desintegration der Struktur der inaktivierten Viren.

Damit ist zum ersten Mal gezeigt, daß eine Formalin-Inaktivierung die intakte Virusstruktur stark beeinflußt.

## Patentansprüche

1. Verfahren zur Inaktivierung von lipidumhüllten Viren ohne die strukturelle Integrität des Virus zu zerstören, **dadurch gekennzeichnet, dass** ein intaktes Virus mit einem nicht-ionischen Detergens aus der Gruppe der Polysorbate über einen Zeitraum inkubiert wird, der ausreicht, um das Virus zu inaktivieren.

2. Verfahren nach Anspruch 1, **dadurch gekennzeichnet, dass** das Virus aus einem Zellkulturüberstand stammt.

3. Vefahren nach Anspruch 1 oder 2, **dadurch gekennzeichnet, dass** das lipidumhüllte Virus ausgewählt ist aus der Gruppe der Retroviren, wie HIV-1, HIV-2, HTLV-I oder HTLV-II, der Flaviviren, wie TBEV oder HCV, der Orthomyxoviren, wie Influenzaviren, der Herpesviren, der Paramyxoviren, wie Mumpsvirus oder Masernvirus, und der Arenaviren, wie Juninvirus oder Lassavirus.

4. Verfahren nach Anspruch 1, **dadurch gekennzeichnet, dass** das Polysorbat Tween® 80, Tween® 40, Tween® 20, Tween® 60 oder eine Kombination derselben ist.

5. Verfahren nach einem der Ansprüche 1 bis 4, **dadurch gekennzeichnet, dass** das nicht-ionische Detergens aus der Gruppe der Polysorbate ein stabilisierendes Detergens ist.

6. Verfahren nach einem der Ansprüche 1 bis 5, **dadurch gekennzeichnet, dass** die Konzentration des nicht-ionischen Detergens aus der Gruppe der Polysorbate zwischen 1% und 20%, vorzugsweise zwischen 10% und 15%, beträgt.

7. Verfahren nach einem der Ansprüche 1 bis 6, **dadurch gekennzeichnet, dass** die Inkubation mit dem nicht-ionischen Detergens für mindestens 10 min erfolgt.

8. Verfahren nach einem der Ansprüche 1 bis 7, **dadurch gekennzeichnet, dass** das nicht-ionische Detergens entfernt wird, gegebenenfalls durch ein chromatographisches Verfahren oder Dialyse.

9. Verfahren zur Herstellung eines inaktivierten Gesamtvirus-Impfstoffes, **dadurch gekennzeichnet, dass** ein gemäß einem der Ansprüche 1 bis 8 inaktiviertes Virus mit einem physiologisch akzeptablen Träger und gegebenenfalls einem Adjuvans versetzt wird.

10. Verwendung einer gemäß einem der Ansprüche 1 bis 9 hergestellten Viruspräparation zur Herstellung eines stabilen Impfstoffes zur Immunisierung von Vertebraten.

## Claims

1. A method of inactivating lipid-enveloped viruses without destructing the structural integrity of the virus, **characterised in that** an intact virus is incubated with a non-ionic detergent from the group of polysorbates for a period of time sufficient to inactivate the virus.

2. A method according to claim 1, **characterised in that** the virus is derived from a cell culture supernatant.

3. A method according to claim 1 or 2, **characterised in that** the lipid-enveloped virus is selected from the group of retroviruses, such as HIV-1, HIV-2, HTLV-I or HTLV-II, flaviviruses, such as TBEV or HCV, orthomixoviruses, such as influenza viruses, herpes viruses, paramyxoviruses, such as mumps virus or measles virus, and arena viruses, such as Junin virus or Lassa virus.

4. A method according to claim 1, **characterised in that** the polysorbate is Tween® 80, Tween® 40, Tween® 20, Tween® 60 or a combination thereof.

5. A method according to any one of claims 1 to 4, **characterised in that** the non-ionic detergent from the group of the polysorbates is a stabilizing detergent.

6. A method according to any one of claims 1 to 5, **characterised in that** the concentration of the non-ionic detergent from the group of polysorbates is between 1% and 20%, preferably between 10% and 15%.

7. A method according to any one of claims 1 to 6, **characterised in that** the incubation with the non-ionic detergent is carried out for at least 10 min.

8. A method according to any one of claims 1 to 7, **characterised in that** the non-ionic detergent is removed, optionally by a chromatographic method or by dialysis.

9. A method of preparing an inactivated whole virus vaccine, **characterised in that** a virus inactivated according to any one of claims 1 to 8 is admixed with a physiologically acceptable carrier and, optionally, with an adjuvant.

10. The use of a virus preparation prepared according to any one of claims 1 to 9 for preparing a stable vaccine for immunizing vertebrates.

## Revendications

1. Procédé d'inactivation de virus enveloppés de lipides sans détruire l'intégrité structurelle du virus, **caractérisé en ce qu'**un virus intact est incubé avec un détergent non-ionique du groupe des polysorbates pendant une durée qui suffit à inactiver le virus.

2. Procédé selon la revendication 1 **caractérisé en ce que** le virus provient d'un surnageant de culture cellulaire.

3. Procédé selon la revendication 1 ou 2 **caractérisé en ce que** le virus enveloppé de lipides est choisi dans le groupe des rétrovirus comme VIH-1, VIH-2, HTLV-I ou HTLV-II, des flavivirus comme TBEV ou VHC, des orthomyxovirus comme les virus grippaux, des virus de l'herpès, des paramyxovirus comme le virus des oreillons ou le virus de la rougeole, et des arénavirus comme le virus Junin ou le virus Lassa.

4. Procédé selon la revendication 1 **caractérisé en ce que** le polysorbate est du Tween® 80, du Tween® 40, du Tween® 20, du Tween® 60 ou une combinaison de ces derniers.

5. Procédé selon l'une quelconque des revendications 1 à 4 **caractérisé en ce que** le détergent non-ionique du groupe des polysorbates est un détergent stabilisant.

6. Procédé selon l'une quelconque des revendications 1 à 5 **caractérisé en ce que** la concentration en détergent non-ionique du groupe des polysorbates varie entre 1 % et 20 %, de préférence entre 10 % et 15 %.

7. Procédé selon l'une quelconque des revendications 1 à 6 **caractérisé en ce que** l'incubation s'effectue avec le détergent non-ionique pendant au moins 10 minutes.

8. Procédé selon l'une quelconque des revendications 1 à 7 **caractérisé en ce que** le détergent non-ionique est éliminé, le cas échéant par un procédé chromatographique ou une dialyse.

9. Procédé de fabrication d'un inoculum de virus entier inactivé **caractérisé en ce qu'**on mélange un virus inactivé selon l'une quelconque des revendications 1 à 8 avec un support physiologiquement acceptable et, le cas échéant, avec un adjuvant.

10. Utilisation d'une préparation virale fabriquée selon l'une quelconque des revendications 1 à 9 pour la fabrication d'un inoculum stable en vue d'immunisation de vertébrés.
